# FASCICULE DE BREVET EUROPEEN

(11) **EP 1 054 638 B1**
(45) Date de publication et mention de la délivrance du brevet: **31.08.2005**
(21) Numéro de dépôt: 99903724.5
(22) Date de dépôt: 27.01.1999
(51) Int. Cl.: A61B 17/70

(54) **STABILISATEUR INTEREPINEUX A FIXER A DES APOPHYSES EPINEUSES DE DEUX VERTEBRES**
AN DEN DORNFORTSÄTZEN ZWEIER WIRBEL ZU BEFESTIGENDE STÜTZVORRICHTUNG
INTERSPINOUS STABILISER TO BE FIXED TO SPINOUS PROCESSES OF TWO VERTEBRAE

(30) Priorité: 10.02.1998 FR 9801528
(43) Date de publication de la demande: 29.11.2000
(73) Titulaire: Dimso (Distribution Medicale du Sud-Ouest), 33610 Cestas (FR); Elberg, Jean François, 75017 Paris (FR)
(72) Inventeur: ELBERG, Jean-François, F-75017 Paris (FR); CLOIX, Erick, F-33000 Bordeaux (FR)
(74) Mandataire: Dossmann, Gérard
(86) Numéro de dépôt international: PCT/FR1999/000154
(87) Numéro de publication internationale: WO 1999/040866

(56) Documents cités:
- EP-A- 0 677 277
- WO-A-94/21185
- WO-A-98/29047
- DE-A- 3 113 142
- FR-A- 2 681 525
- FR-A- 2 717 675
- FR-A- 2 722 980

## Description

L'invention concerne les stabilisateurs interépineux à fixer à deux vertèbres respectives.

On connaît un stabilisateur de ce type, comportant deux organes d'ancrage à fixer aux pédicules vertébraux de deux vertèbres, et un corps rigide reliant les deux organes l'un à l'autre. En cas de dégénérescence du disque, ce stabilisateur permet d'immobiliser rigidement les deux vertèbres l'une par rapport à l'autre et ainsi de soulager le disque intervertébral associé. Toutefois, ce stabilisateur ne donne pas entière satisfaction. En effet, l'opération pour mettre en place ce stabilisateur nécessite d'atteindre les pédicules vertébraux, voire le disque. Il faut donc pénétrer profondément dans le corps du patient, ce qui alourdit l'opération. De plus, les deux vertèbres étant totalement immobilisées l'une par rapport à l'autre, le disque n'est plus sollicité et sa dégénérescence se poursuit. En outre, les organes d'ancrage aux pédicules fragilisent ces derniers et entraînent une modification partielle des apophyses articulaires. Enfin, la mise en place des organes d'ancrage requiert une visée délicate dans les pédicules pour ne pas sortir de ceux-ci et par exemple toucher la dure-mère.

On connaît par ailleurs du document FR-2 722 980 un stabilisateur interépineux ayant des organes d'ancrage aux apophyses épineuses reliées l'un à l'autre par un ressort à lame en forme de « U » dans un plan perpendiculaire à la génératrice géométrique de la lame. e stabilisateur est apte à être fixé au rachis de sorte que la génératrice est parallèle à la direction droite-gauche par référence au corps du patient, la base du « U » s'étendant du côté des corps vertébraux en appui contre l'une des vertèbres. Le stabilisateur est ainsi entièrement logé entre les apophyses. Il s'ensuit que le stabilisateur a nécessairement une très petite taille. Or, cela complique sa réalisation ou bien cela oblige à lui donner une forme très simple le rendant fortement rigide, ce qui génère de nouveaux risques de dégénérescence du disque comme précité.

Un but de l'invention est de fournir un stabilisateur d'un type différent, et notamment facile à fabriquer et pouvant avoir une faible rigidité.

En vue de la réalisation de ce but, on prévoit selon l'invention un stabilisateur interépineux comportant deux organes d'ancrage à des apophyses épineuses de deux vertèbres respectives, et un corps s'étendant suivant une direction d'alignement des organes, le corps étant compressible suivant la direction d'alignement sous l'effet d'une sollicitation à partir d'une configuration donnée, le corps étant adapté à recouvrer spontanément la configuration donnée après que la sollicitation a cessé, le corps comprenant un ressort à lame ayant une génératrice géométrique, dans lequel les organes d'ancrage sont aptes à fixer le stabilisateur aux apophyses de sorte que la génératrice s'étend sensiblement d'avant en arrière par référence au corps du patient.

Ainsi, le stabilisateur autorise une certaine mobilité des deux vertèbres l'une par rapport à l'autre en reproduisant partiellement la biomécanique d'un disque intervertébral sain. De plus, le disque continue à être partiellement sollicité même si le stabilisateur le soulage d'une grande partie des sollicitations pesant d'ordinaire sur lui. On peut ainsi ralentir voire arrêter la dégénérescence du disque. Le stabilisateur permet de garder l'intégrité de l'articulation tripode de l'unité vertébrale : le disque et les deux articulaires postérieures ainsi que les connexions associées au niveau d'une vertèbre que sont les pédicules et les lames. La mise en place du stabilisateur sur les apophyses épineuses est simple à réaliser. En outre, on est assuré de conserver l'intégrité de la protection de la dure-mère.

De plus, l'orientation de la génératrice suivant la direction avant-arrière permet d'étendre le stabilisateur latéralement au-delà des apophyses. On peut donc accroître son volume pour le rendre à la fois plus facile à fabriquer et si besoin moins rigide en vue de limiter les risques de dégénérescence du disque.

Le stabilisateur selon l'invention pourra en outre présenter une ou plusieurs des caractéristiques suivantes :
- le corps comprend deux parties de ressort à lame s'étendant en parallèle l'une de l'autre suivant la direction d'alignement ;
- chaque partie forme au moins un « U » dans un plan perpendiculaire à la génératrice ;
- le corps comprend au moins une partie de ressort à lame formant, dans un plan perpendiculaire à la génératrice, au moins deux « U » successifs orientés en sens contraires en alternance l'un par rapport à l'autre ;
- le ressort présente au moins deux tronçons d'épaisseurs différentes ;
- le corps comporte deux ressorts à lame en appui l'un sur l'autre ;
- le corps comporte un ressort à lame conformé en une boucle fermée ;
- la boucle a une forme en ellipse ;
- le ressort a une épaisseur plus grande au voisinage d'un grand axe de la boucle qu'au voisinage d'un petit axe de la boucle ;
- le corps comporte au moins un élément en matériau viscoélastique ;
- l'élément est disposé à l'intérieur de la boucle ;
- le corps comporte deux éléments en matériau viscoélastique disposés au voisinage de deux extrémités respectives d'un grand axe de la boucle ; et
- le ou chaque élément a une face cylindrique en contact avec une face du ressort.

D'autres caractéristiques et avantages de l'invention apparaîtront encore dans la description suivante de deux modes préférés de réalisation et de variantes donnés à titre d'exemples non limitatifs. Aux dessins annexés :
- la figure 1 est une vue d'un premier mode préféré de réalisation du stabilisateur selon l'invention ;
- les figures 2 et 3 sont des vues respectives de deux variantes de ce premier mode de réalisation ;
- la figure 4 est une vue en élévation d'un deuxième mode préféré de réalisation du stabilisateur de l'invention ;
- la figure 5 est une vue en perspective du stabilisateur de la figure 4 une fois installé ;

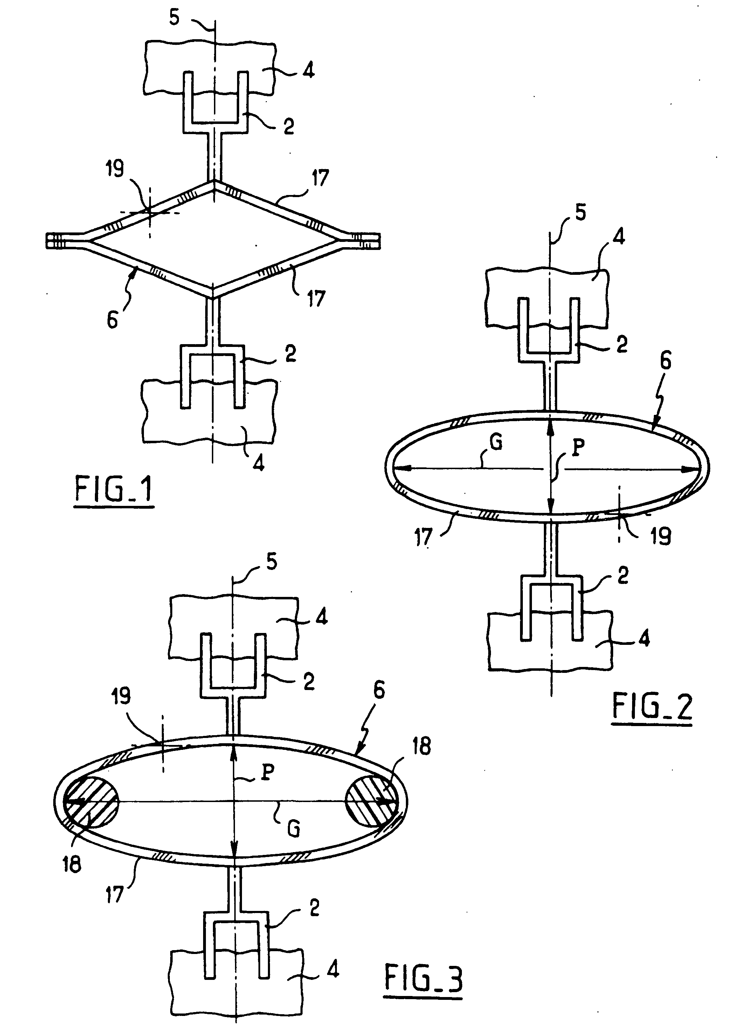

En référence à la figure 1, dans un premier mode de réalisation, le stabilisateur selon l'invention comporte deux organes d'ancrage 2 d'un type connu en soi adaptés à être fixés rigidement aux apophyses épineuses de deux vertèbres adjacentes 4 respectives. Ces organes d'ancrage sont par exemple du type de ceux du document FR-2 722 980. Le stabilisateur comporte un corps 6 s'étendant suivant une direction d'alignement 5 des organes d'ancrage 2, entre ceux-ci, et reliant les organes d'ancrage. Le corps 6 est compressible suivant la direction 5 sous l'effet d'une sollicitation tendant à rapprocher les deux apophyses l'une de l'autre. On suppose que le corps 6 est compressé à partir d'une configuration de départ donnée. Lorsque cesse la sollicitation, le corps 6 recouvre spontanément sa configuration de départ. Les deux organes 2 sont également mobiles en rotation l'un par rapport à l'autre autour d'un point de rotation passant par la direction 5. Sous l'effet d'une sollicitation adaptée, on peut ainsi leur donner momentanément une inclinaison relative, les organes 2 redevenant parallèles entre eux lorsque disparaît cette sollicitation.

Le corps 6 comporte deux ressorts à lame 17 identiques entre eux, chacun de forme plate rectiligne allongée. Une partie médiane de chaque ressort 17 est fixée rigidement par une face à une extrémité de l'un des organes d'ancrage 2 respectifs. Les extrémités des ressorts 17 sont fixées l'une à l'autre et sont en appui l'une sur l'autre. Les deux ressorts 17 présentent un pli à leur partie médiane de sorte qu'ils forment un losange. Les ressorts à lames ont une génératrice géométrique 19. Les organes d'ancrage sont positionnés de sorte qu'une fois le stabilisateur en place sur le patient, la génératrice 19 s'étend d'avant en arrière par référence au corps du patient et perpendiculairement au plan de la feuille sur la figure 1. Seule la tranche des ressorts 17 est visible sur la figure 1. Lorsqu'une sollicitation suivant la direction 5 tend à rapprocher les deux organes d'ancrage 2 l'un de l'autre, le losange se déforme sensiblement élastiquement pour tendre à s'aplatir. Lorsque la sollicitation cesse, grâce à la raideur des ressorts 17, le corps 6 recouvre sa configuration de départ. Ce mode de réalisation autorise de modifier l'inclinaison relative des organes d'ancrage 2 sous l'effet d'une sollicitation adaptée, cette inclinaison disparaissant par élasticité en même temps que la sollicitation qui l'a fait naître.

Dans la variante de la figure 2, le corps 6 comprend un unique ressort à lame 17 courbé sur lui-même pour être conformé en une boucle fermée ici en forme d'ellipse. Le ressort 17 est fixé rigidement aux organes d'ancrage 2, entre ceux-ci, de sorte que la direction 5 constitue le petit axe P de l'ellipse. La génératrice 19 est orientée de la même façon que dans le mode de réalisation de la figure 1. Ce stabilisateur fonctionne essentiellement de la même façon que celui de la figure 1. Avantageusement, la lame du ressort 17 pourra présenter des épaisseurs différentes en différents endroits de la lame. Par exemple, la lame aura une épaisseur plus importante au voisinage du grand axe G de l'ellipse qu'au voisinage du petit axe P de l'ellipse. Ainsi, on paramètre la raideur du ressort 17 en fonction de la partie concernée de la lame. On obtient notamment une déformation non uniforme des différentes parties du ressort sous l'effet d'une sollicitation suivant la direction 5.

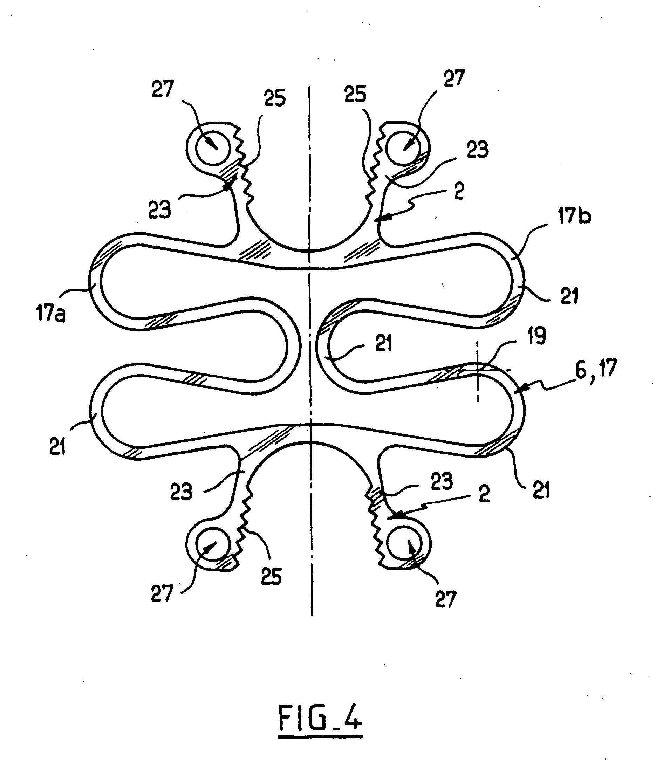

Dans la variante de la figure 3, le corps 6 comporte un ressort 17 en ellipse et en outre deux noyaux 18 en un matériau viscoélastique tel que du polyuréthanne ou du silicone. Ces noyaux 18 ont chacun une forme cylindrique. Ils sont disposés à l'intérieur de l'ellipse, aux extrémités du grand axe G, avec leurs axes perpendiculaires aux axes P, G de l'ellipse et parallèles à la génératrice 19 du ressort et leur face cylindrique en contact avec la face interne de la lame. Avantageusement, chaque noyau 18 a un rayon inférieur ou égal au plus petit rayon de courbure de la lame, au niveau du grand axe G. Les noyaux 18 modifient le comportement du corps 6 lors de sa compression et de sa détente.

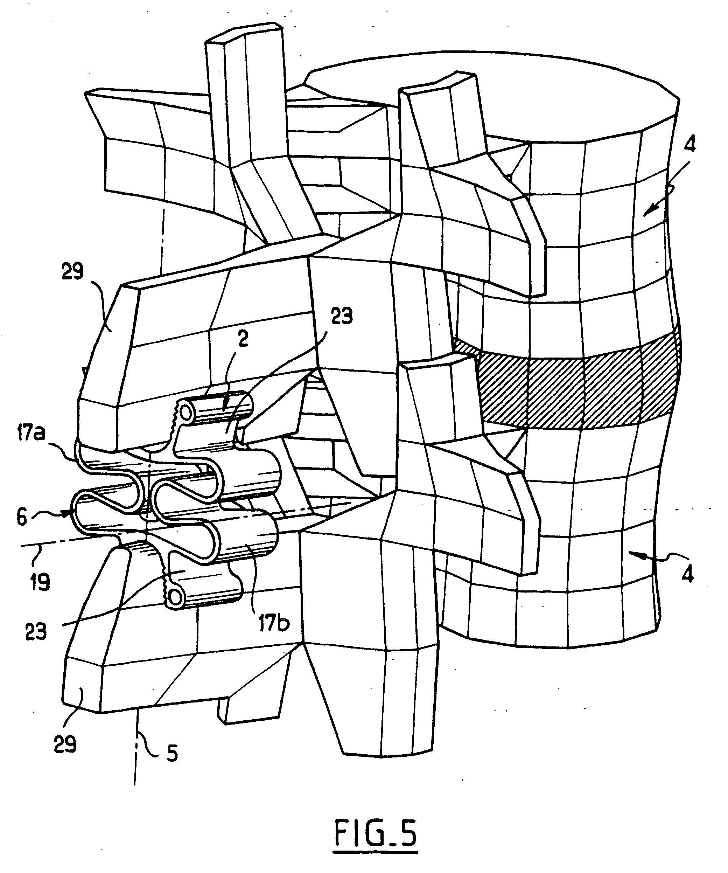

Dans le deuxième mode de réalisation illustré aux figures 4 et 5, le corps 6 comporte une fois encore un ressort à lame 17 conformé en une boucle fermée d'un seul tenant avec les organes d'ancrage 2. Le stabilisateur est réalisé en titane ou alliage de titane. Comme dans les stabilisateurs des figures 1, 2 et 3, le ressort définit deux parties de ressort à lame 17a, 17b s'étendant en parallèle l'une de l'autre suivant la direction d'alignement 5. La génératrice 19, visible également sur la figure 5, s'étend encore d'avant en arrière.

Les deux parties du ressort 17a, 17b sont symétriques l'une de l'autre par rapport à un plan médian passant par l'axe 5. Chaque partie de ressort forme, dans un plan perpendiculaire à la génératrice 19, plusieurs « U » successifs orientés en sens contraires les uns aux autres en alternance. Sur chaque partie 17a, 17b, les « U » sont ici au nombre de trois. Les « U » les plus proches des organes d'ancrage 2 ont leur base 21 située vers l'extérieur du stabilisateur, alors que le « U » médian de chaque partie a sa base 21 vers l'intérieur du stabilisateur. Chaque partie 17a, 17b a donc la forme d'une ondulation ou d'un zigzag. Plus précisément, cette forme est ici généralement celle d'un « M » renversé.

Chacun des organes d'ancrage 2 comprend ici deux mors 23 symétriques l'un de l'autre par rapport au plan médian, de forme générale plate et ayant une génératrice parallèle à la génératrice 19. Les deux mors 23 s'étendent en regard l'un de l'autre. Leurs faces en regard présentent des dents profilées 25. Chaque mors présente un conduit 27 d'axe parallèle à la génératrice 19 pour l'introduction d'un outil de manoeuvre du mors. Les bases des mors s'étendent à distance l'une de l'autre à partir d'une extrémité du ressort 17. Les deux mors sont mobiles élastiquement l'un par rapport à l'autre. Au repos, ils s'étendent de façon divergente à partir de leur base.

L'ensemble du stabilisateur est profilé suivant un axe parallèle à la génératrice 19, le profil étant représenté à la figure 4.

Pour mettre en place le stabilisateur, on sollicite les mors 23 de chaque organe d'ancrage 2 en éloignement l'un de l'autre, au moyen d'outils introduits dans les conduits 27. Puis on place le stabilisateur comme sur la figure 5 de sorte que chaque apophyse 29 est entre les mors 23 respectifs. Puis on relâche les mors pour qu'ils pincent les apophyses et s'ancrent à celles-ci au moyen de leurs dents 25.

De même que dans les précédents stabilisateurs des figures 1 à 3, les parties de ressort à lame 17a, 17b s'étendent latéralement au-delà des apophyses 29, comme illustré à la figure 5. On peut les configurer pour leur donner une faible raideur. La fabrication du stabilisateur est effectuée par électro-érosion d'une masse de métal, cette fabrication étant particulièrement simple grâce à la forme profilée du stabilisateur. De même que les stabilisateurs des figures 1 à 3, ce stabilisateur offre une raideur assez faible pour des flexions latérales du corps c'est-à-dire autour d'un axe de flexion parallèle à la génératrice 19. Il offre une raideur importante pour des flexions du corps d'avant en arrière c'est-à-dire autour d'un axe perpendiculaire à la direction 5 et à la génératrice 19. On peut facilement modifier la forme du ressort pour accroître ou réduire l'une au moins de ces raideurs, indépendamment du volume disponible entre les apophyses 29.

## Revendications

1. Stabilisateur interépineux comportant deux organes d'ancrage (2) à des apophyses épineuses de deux vertèbres respectives (4), et un corps (6) s'étendant suivant une direction d'alignement (5) des organes (2), le corps (6) étant compressible suivant la direction d'alignement (5) sous l'effet d'une sollicitation à partir d'une configuration donnée, le corps étant adapté à recouvrer spontanément la configuration donnée après que la sollicitation a cessé, le corps comprenant un ressort à lame (17) ayant une génératrice géométrique (19), **caractérisé en ce que** les organes d'ancrage (2) sont aptes à fixer le stabilisateur aux apophyses de sorte que la génératrice (19) s'étend sensiblement d'avant en arrière par référence au corps du patient en état implanté du statilisateur.

2. Stabilisateur selon la revendication 1, **caractérisé en ce que** le corps (6) comprend deux parties de ressort à lame (17a, 17b) s'étendant en parallèle l'une de l'autre suivant la direction d'alignement (5).

3. Stabilisateur selon la revendication 2, **caractérisé en ce que** chaque partie (17a, 17b) forme au moins un « U » dans un plan perpendiculaire à la génératrice (19).

4. Stabilisateur selon l'une quelconque des revendications 1 à 3, **caractérisé en ce que** le corps (6) comprend au moins une partie de ressort à lame (17a, 17b) formant, dans un plan perpendiculaire à la génératrice (19), au moins deux « U » successifs orientés en sens contraires en alternance l'un par rapport à l'autre.

5. Stabilisateur selon l'une quelconque des revendications 1 à 4, **caractérisé en ce que** le ressort à lame (17) est conformé en une boucle fermée.

6. Stabilisateur selon la revendication 5, **caractérisé en ce que** la boucle a une forme en ellipse.

7. Stabilisateur selon l'une quelconque des revendications 1 à 6, **caractérisé en ce que** le corps (6) comporte au moins un élément (18) en matériau viscoélastique.

8. Stabilisateur selon l'une quelconque des revendications 1 à 4 ou selon la revendication 7, **caractérisé en ce que** le corps (6) comporte deux ressorts à lame (17) en appui l'un sur l'autre.

9. Stabilisateur selon l'une quelconque des revendications 1 à 8, **caractérisé en ce que** le ressort (17) présente au moins deux tronçons d'épaisseurs différentes.

10. Stabilisateur selon l'une quelconque des revendications 1 à 9, **caractérisé en ce que** le stabilisateur comporte au moins deux corps (6) disposés mutuellement en parallèle suivant la direction d'alignement (5).

11. Stabilisateur selon l'une quelconque des revendications 1 à 10, **caractérisé en ce qu'**au moins l'un des organes d'ancrage (2) comprend deux mors (23) dentés mobiles élastiquement en direction l'un de l'autre pour former une pince.

## Patentansprüche

1. Interspinaler Stabilisator mit zwei Einrichtungen zur Verankerung (2) an den Dornfortsätzen von jeweils zwei Wirbeln (4) und mit einem Körper (6), der sich in Ausrichtungslinie (5) der Verankerungseinrichtungen (2) dehnt, wobei der Körper (6) in der Ausrichtungslinie (5) komprimierbar ist, wenn eine Beanspruchung aus einer gegebenen Konfiguration heraus erfolgt, und wobei der Körper fähig ist, die gegebene Konfiguration spontan wieder einzunehmen, nachdem die Beanspruchung aufgehört hat, und wobei der Körper eine Blattfeder (17) mit einer Mantellinie (19) enthält, **dadurch gekennzeichnet, dass** die Verankerungseinrichtungen (2) den Körper so an den Dornfortsätzen fixieren, dass die Mantellinie (19) sich im Bezug zum Körper des Patienten deutlich von vorne nach hinten ausdehnt, wenn der Stabilisator implantiert ist.

2. Stabilisator nach Anspruch 1, **dadurch gekennzeichnet, dass** der Körper (6) zwei Blattfederkomponenten (17a, 17b) enthält, die sich parallel zueinander in der Ausrichtungslinie (5) erstrecken.

3. Stabilisator nach Anspruch 2, **dadurch gekennzeichnet, dass** jede Komponente (17a, 17b) in einer senkrecht zur Mantellinie (19) stehenden Ebene mindestens ein "U" bildet.

4. Stabilisator nach irgendeinem der Ansprüche 1 bis 3, **dadurch gekennzeichnet, dass** der Körper (6) mindestens eine Blattfederkomponente (17a, 17b) enthält, die in einer senkrecht zur Mantellinie (19) stehenden Ebene mindestens zwei nacheinanderstehende "U" bildet, die abwechselnd in umgekehrter Richtung zueinander ausgerichtet sind.

5. Stabilisator nach irgendeinem der Ansprüche 1 bis 4, **dadurch gekennzeichnet dass** die Blattfeder (17) eine geschlossene Schleife bildet.

6. Stabilisator nach Anspruch 5, **dadurch gekennzeichnet, dass** die Schleife ellipsenförmig ist.

7. Stabilisator nach irgendeinem der Ansprüche 1 bis 6, **dadurch gekennzeichnet, dass** der Körper (6) mindestens ein Element (18) aus viskoelastischem Material enthält.

8. Stabilisator nach irgendeinem der Ansprüche 1 bis 4 oder nach Anspruch 7, **dadurch gekennzeichnet, dass** der Körper (6) zwei Blattfedern enthält (17), die aufeinander aufliegen.

9. Stabilisator nach irgendeinem der Ansprüche 1 bis 8, **dadurch gekennzeichnet, dass** die Feder (17) mindestens zwei Teilstücke verschiedener Stärke enthält.

10. Stabilisator nach irgendeinem der Ansprüche 1 bis 9, **dadurch gekennzeichnet, dass** der Stabilisator mindestens zwei Körper (6) enthält, die in der Ausrichtungslinie (5) parallel zueinander angeordnet sind.

11. Stabilisator nach irgendeinem der Ansprüche 1 bis 10, **dadurch gekennzeichnet, dass** mindestens eine der Verankerungseinrichtungen (2) zwei zueinander elastisch mobile gezahnte Backen enthält, die eine Zange bilden.

## Claims

1. Interspinous stabilizer comprising two anchorage organs (2) to the spinous apophysis of two respective vertebrae (4) and a body (6) extending along the organs (2) line direction (5), being the body compressible along the line direction (5) under the influence of a load, from a given configuration, and being the body adapted to spontaneously recover the given configuration, once the load has stopped, the body comprising a laminated spring (17) having a geometric generator (19), **characterized in that** the anchorage organs (2) are able to fix the stabilizer to the apophysis so that the generator (19) appreciably extends from the front to the rear, with regard to the patient's body, being implanted in the stabilizer.

2. Stabilizer according to the claim 1, **characterized in that** the body (6) consists of two parts of laminated spring (17a and 17b) extending in parallel, one to the other, along the line direction (5).

3. Stabilizer according to claim 2, **characterized in that** each part (17a and 17b) makes up, at least, a "U", in a plan perpendicular to the generator (19).

4. Stabilizer according to any of the claims 1 to 3, **characterized in that** the body (6) comprises at least a part of laminated spring (17a, 17b), making up, in a plan perpendicular to the generator (19), at least two successive "U", oriented in opposite direction, alternately one compared with the other.

5. Stabilizer according to any of the claims 1 to 4, **characterized in that** the laminated spring is configured in a closed buckle.

6. Stabilizer according to claim 5, **characterized in that** the buckle is ellipse shaped.

7. Stabilizer according to any of the claims 1 to 6, **characterized in that** the body (6) includes at least one element (18) made up of a viscoelastic material.

8. Stabilizer according to any of the claims 1 to 4,or according to claim 7, **characterized in that** the body (6), includes two laminated springs (17), one supporting the other.

9. Stabilizer according to any of the claims 1 to 8, **characterized in that** the spring (17) has at least two sections which thickness is different.

10. Stabilizer according to any of the claims 1 to 9, **characterized in that** the stabilizer comprises at least two bodies (6), set out parallel one to the other, along the direction line (5).

11. Stabilizer according to any of the claims 1 to 10, **characterized in that** at least one of the anchorage organs (2) includes two elastically mobile cogged jaws, oriented one toward the other, in order to form pincers.
